# FASCICULE DE BREVET EUROPEEN

(11) **EP 2 612 851 B1**
(45) Date de publication et mention de la délivrance du brevet: **06.07.2016**
(21) Numéro de dépôt: 13150305.4
(22) Date de dépôt: 04.01.2013
(51) Int. Cl.: C07D 209/42

(54) **Procédé de préparation du sel de l-arginine du périndopril**
Herstellungsverfahren des Argininsalzes von Perindopril
Process for the preparation of the L-arginine salt of perindopril

(30) Priorité: 05.01.2012 FR 1200034
(43) Date de publication de la demande: 10.07.2013
(73) Titulaire: Les Laboratoires Servier, 92284 Suresnes (FR)
(72) Inventeur: Linol, Julie, 76770 MALAUNAY (FR); Laurent, Stéphane, 76190 VALLIQUERVILLE (FR); Grenier, Arnaud, 76110 BREAUTE (FR); Mathieu, Sébastien, 76290 MONTIVILLERS (FR)

(56) Documents cités:
- EP-A1- 1 864 973
- EP-A1- 2 161 257
- EP-A2- 1 279 665
- WO-A1-2007/099217
- WO-A2-2007/099216
- WO-A2-2009/157018

## Description

La présente invention concerne un procédé de préparation du sel de L-arginine du périndopril de formule (I) :

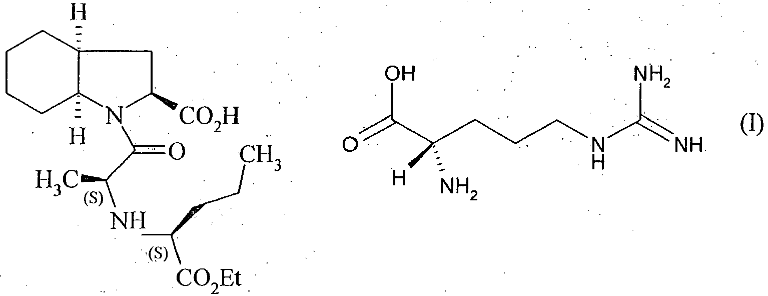

Le périndopril, ainsi que ses sels pharmaceutiquement acceptables, et plus particulièrement son sel de L-arginine, possèdent des propriétés pharmacologiques intéressantes.

Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.

Ces deux actions contribuent aux effets bénéfiques du périndopril dans les maladies cardiovasculaires, tout particulièrement l'hypertension artérielle, l'insuffisance cardiaque et la maladie coronaire stable.

Le périndopril, sa préparation et son utilisation en thérapeutique ont été décrits dans le brevet européen EP 0 049 658.

Le sel de L-arginine du périndopril a été décrit pour la première fois dans le brevet européen EP 1 354 873.

Les formes cristallines alpha et bêta du sel de L-arginine du périndopril ont été décrites dans les brevets européens EP 1 989 182 et EP 2 016 051.

La forme cristalline gamma du sel de L-arginine du périndopril a été décrite dans la demande de brevet WO 2009/157018.

Compte tenu de l'intérêt pharmaceutique du périndopril L-arginine, il était primordial de l'obtenir avec un bon rendement et une excellente pureté.

Plus particulièrement, le problème était à la fois de trouver des conditions dans lesquelles la salification du périndopril par la L-arginine se fait correctement, et d'isoler facilement le sel de L-arginine du périndopril du milieu réactionnel.

En effet, la plupart des expériences menées par le Demandeur pour obtenir le sel de L-arginine du périndopril à partir de périndopril et de L-arginine ont conduit à un produit d'aspect gélatineux, très difficile à processer par la suite.

Le brevet EP 1 279 665 décrit une méthode d'obtention de sels de périndopril, plus spécifiquement le sel de tert-butylamine. La méthode décrite permet de coupler la partie N-[1-(S)-éthoxycarbonyl-butyl]-(S)-alanine à la partie (2S, 3aS, 7aS)-2-carboxyperhydroindole en évitant les impuretés de cyclisation qui résultent habituellement du couplage peptidique. Le sel de tert-butylamine du périndopril est ainsi obtenu dans l'Exemple 3 de EP 1 279 665 avec un bon rendement (80%) et une excellente pureté (99%).

Le Demandeur a appliqué le procédé décrit dans l'Exemple 3 de EP 1 279 665 à la préparation du sel de L-arginine du périndopril. Cependant, le remplacement de la tert-butylamine par la (L)-arginine en suivant par ailleurs le mode opératoire de EP 1 279 665 n'a pas permis d'obtenir le sel de L-arginine du périndopril avec un bon rendement (voir Exemple comparatif A).

De façon surprenante, lorsque la salification est cette fois effectuée dans un système de solvants choisi parmi acétonitrile / diméthylsulfoxyde, acétate d'éthyle / diméthylsulfoxyde et acétonitrile / diméthylsulfoxyde / toluène, le sel de L-arginine du périndopril est alors obtenu avec un bon rendement et une excellente pureté, et l'isolement est grandement facilité.

Plus spécifiquement, la présente invention concerne un procédé de préparation du sel de L-arginine du périndopril par réaction entre le périndopril et la L-arginine dans un système de solvants choisi parmi :
un mélange binaire d'acétonitrile et de diméthylsulfoxyde,
un mélange binaire d'acétate d'éthyle et de diméthylsulfoxyde,
un mélange ternaire d'acétonitrile, de diméthylsulfoxyde et de toluène,
à une température de 10 à 100°C, préférentiellement de 40 à 80°C, suivie de l'isolement par filtration du sel de L-arginine ainsi obtenu.

Selon un mode de réalisation de la présente invention, le périndopril (acide libre) utilisé dans la réaction est obtenu par réaction de la N-[1-(S)-éthoxycarbonyl-butyl]-(S)-alanine de formule (II) : 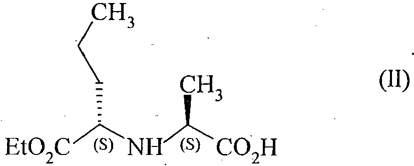 avec un agent d'activation, préférentiellement le N, N'-carbonyldiimidazole, le phosgène, le triphosgène, le (1,1'-carbonyldi(1,2,4-triazole) ou le di(N-succinimidyl)carbonate,
dans un solvant organique ou système de solvants organiques, préférentiellement l'acétonitrile, l'acétate d'éthyle ou le dichlorométhane,
à une température de - 20°C à 80°C, préférentiellement de -10 à 40°C, suivie de la réaction du composé intermédiaire de formule (III) ainsi obtenu : 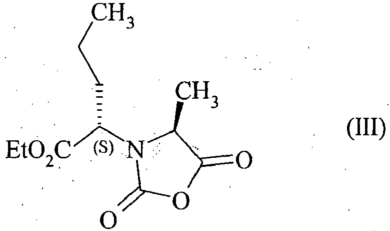 avec le (2S, 3aS, 7aS)-2-carboxyperhydroindole, à une température de 0°C à 80°C, préférentiellement de 5 à 40°C.

Par "agent d'activation", on entend un composé de formule X₂C=O dans laquelle X représente un groupe partant tel que, par exemple, un atome d'halogène ou un groupement tosylate, mésylate, imidazolyle, 1,2,4-triazolyle, succinimidyle ou alkoxy éventuellement substitué.

Lorsque l'agent d'activation est le N, N'-carbonyldiimidazole, la quantité de N, N'-carbonyldiimidazole est préférentiellement comprise entre 0,8 et 1,2 mole par mole de N-[1-(S)-éthoxycarbonyl-butyl]-(S)-alanine.

La quantité de (2S, 3aS, 7aS)-2-carboxyperhydroindole est préférentiellement comprise entre 0,8 et 1,2 mole par mole de N-[1-(S)-éthoxycarbonyl-butyl]-(S)-alanine.

Selon un autre mode de réalisation de la présente invention, le périndopril (acide libre) utilisé dans la réaction est obtenu par désalification du périndopril tert-butylamine par action d'un acide.

Par désalification du périndopril tert-butylamine, on entend retour au périndopril sous forme d'acide libre.

Les exemples suivants illustrent l'invention.

Ces exemples ne conduisent pas tous à la forme cristalline delta pure.

### Abréviations :

- CDT: (1,1'-carbonylDi(1,2,4-Triazole))
- DMSO: Diméthylsulfoxyde
- DSC: (Di(N-Succinimidyl)Carbonate)
- HPLC: High Performance Liquid Chromatography (Chromatographie en phase liquide à haute performance)
- m/m: rapport exprimé en masse/masse

Les filtrations sont exprimées de façon standard, en kg de jus filtré par heure et par m² de surface filtrante.

### EXEMPLE 1 : Sel de L-arginine du périndopril - à partir du périndopril (acide libre), dans un mélange binaire acétonitrile/DMSO 25/75 sans amorce

Le périndopril (12,5 g, 1 éq.) et la L-arginine (5,32 g - 0,9 éq) sont mis en suspension dans un mélange d'acétonitrile (20 g, d = 0,787) et de DMSO (61 g, d = 1,100). Le milieu réactionnel est chauffé à 50°C pendant une nuit. Le produit est ensuite isolé par filtration sur fritté. Le gâteau est lavé et séché.

Le périndopril arginine (14,5g) est obtenu avec un rendement de 79 % par rapport au périndopril. La phase cristalline isolée est la phase delta. La qualité HPLC du produit isolé est supérieure à 99,0 %.
Le débit de filtration des jus mères est de l'ordre de 6000 kg/h/m².

Le sel de L-arginine du périndopril ainsi obtenu est sous la forme cristalline delta. Celle-ci a le diagramme de diffraction X sur poudre suivant, mesuré sur un diffractomètre à anticathode de cuivre et exprimé en termes de distance inter-réticulaire d, d'angle de Bragg 2 thêta et d'intensité relative, exprimée en pourcentage par rapport à la raie la plus intense :

| **Angle 2 thêta (°)** | **Distance interréticulaire d [Å]** | **Intensité relative [%]** |
|---|---|---|
| 4,34 | 20,37 | 66,2 |
| 5,57 | 15,86 | 5,2 |
| 11,04 | 8,02 | 57,5 |
| 11,15 | 7,94 | 47,5 |
| 11,87 | 7,454 | 35,0 |
| 12,47 | 7,09 | 17,9 |
| 13,21 | 6,70 | 33,6 |
| 14,06 | 6,30 | 6,6 |
| 14,64 | 6,05 | 31,8 |
| 16,03 | 5,53 | 17,5 |
| 17,11 | 5,18 | 5,6 |
| 18,27 | 4,85 | 4,1 |
| 19,23 | 4,61 | 100 |
| 19,44 | 4,57 | 17,8 |
| 20,04 | 4,43 | 13,6 |
| 21,11 | 4,21 | 3,7 |
| 21,93 | 4,05 | 23,0 |
| 22,20 | 4,00 | 16,9 |
| 22,61 | 3,93 | 21,2 |
| 23,21 | 3,83 | 4,5 |
| 24,30 | 3,66 | 2,3 |
| 25,09 | 3,55 | 9,4 |
| 25,95 | 3,43 | 1,7 |
| 29,54 | 3,02 | 4,2 |

Chaque raie est considérée avoir une précision de ± 0,2° en 2-thêta.

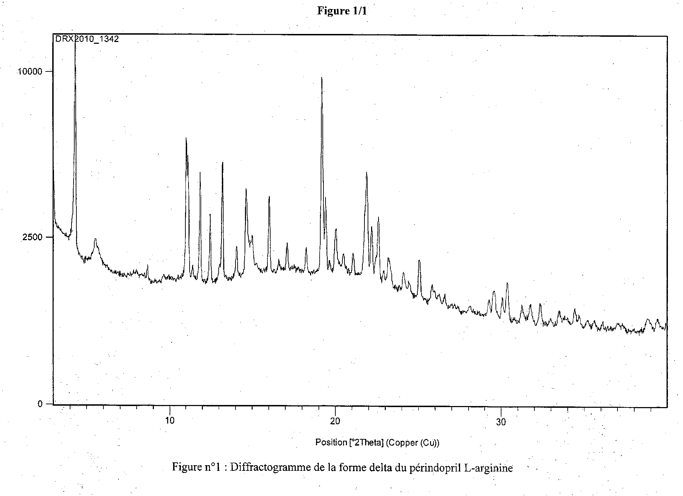

Figure 1 : Diffractogramme de la forme delta du périndopril L-arginine.

### EXEMPLE 2 : Sel de L-arginine du périndopril - à partir du périndopril, (acide libre), dans un mélange binaire acétonitrile/DMSO 25/75 avec amorce

Le périndopril (100 g, 1 éq.) et la L-arginine (42,6 g, 0,9 éq.) sont mis en suspension dans un mélange d'acétonitrile (220 g, d = 0,787) et de diméthylsulfoxyde (630 g, d = 1,100). Le milieu réactionnel est chauffé à 70°C pendant 3h et amorcé avec 2 % de phase delta puis refroidi à 40°C en 1h. Le milieu est conservé sous agitation à 40°C pendant 18h puis refroidi à 20°C en 1h. Le produit est ensuite isolé par filtration. Le gâteau est lavé et séché.
Le périndopril (L)-arginine (119 g) est obtenu avec un rendement de 79 % (amorce déduite) par rapport au périndopril. La qualité HPLC du produit isolé est supérieure à 99,0 %.
Le débit de filtration des jus mères se situe environ à 6000 kg/h/m².

### EXEMPLE 3 : Procédure générale de fabrication du périndopril (acide libre) à partir de (2S, 3aS, 7aS)-2-carboxy-perhydroindole et de N-[1-(S)-éthoxy-carbonyl-butyl]-(S)-alanine par activation avec le N, N'-carbonyl-diimidazole.

La N-[1-(S)-éthoxycarbonyl-butyl]-(S)-alanine (65 g, 1 éq.) et le N, N'-carbonyldiimidazole (48 g, 1 éq.) sont chargés puis de l'acétonitrile (500 g) est additionné. Le milieu réactionnel est ensuite agité à une température inférieure à +10°C pendant 3h.
Le milieu réactionnel est coulé sur le (2S, 3aS, 7aS)-2-carboxyperhydroindole (50 g, 1 éq.), une nouvelle charge d'acétonitrile (80 g) est utilisée pour rincer l'équipement.
Le milieu réactionnel est ensuite agité pendant 5 h à une température inférieure à +10°C puis clarifié sur un filtre pour obtenir une solution limpide.

### EXEMPLE 4 : Sel de L-arginine du périndopril - à partir de (2S, 3aS, 7aS)-2-carboxy-perhydroindole, dans un mélange binaire acétonitrile/DMSO 50/50 avec amorce

Le périndopril L-arginine (110 g) est obtenu par coulée de la solution dans l'acétonitrile de périndopril (100 g de produit) synthétisé selon la procédure générale de l'Exemple 3 sur une suspension de L-arginine (44,3 g, 0,85éq.) dans le DMSO (540 g, d = 1,100) à 50°C, le milieu réactionnel est amorcé avec 2% de la forme cristalline delta (composé de l'Exemple 1). Le milieu est maintenu sous agitation à 50°C pendant 15h puis refroidi à 20°C avec une pente de 0,5°C/min. La suspension est filtrée en cellule de filtration. Le rendement est de 75% (amorce déduite) par rapport au périndopril engagé. La qualité du produit isolé est supérieure à 99,0 % par HPLC.
Le débit de filtration des jus mères est d'environ 5000 kg/h/m².

### EXEMPLE 5 : Sel de L-arginine du périndopril - à partir de (2S, 3aS, 7aS)-2-carboxy-perhydroindole, dans un mélange binaire acétonitrile/DMSO 75/25 avec amorce

Le périndopril L-arginine (42 g) est obtenu par coulée de la solution dans l'acétonitrile de périndopril (38 g de produit) synthétisé selon la procédure générale de l'Exemple 3 par ajout de L-arginine (17 g, 0,85 éq.) en suspension dans le diméthylsulfoxyde (78 g) à la température de 40°C et après amorçage avec 4 % massique de périndopril L-arginine sous forme delta (composé de l'Exemple 1). La filtration a lieu à 40°C en cellule de filtration.
Le rendement est de 73 % (amorce déduite) par rapport au périndopril engagé. La qualité du produit isolé est supérieure à 99,0 % par HPLC.
Le débit de filtration des jus mères est d'environ 5700 kg/h/m².

### EXEMPLE 6 : Sel de L-arginine du périndopril - à partir de (2S, 3aS, 7aS)-2-carboxy-perhydroindole et de triphosgène.

La N-[1-(S)-éthoxycarbonyl-butyl]-(S)-alanine (20 g, 1 éq.), Na₂HPO₄,12 H₂O (43 g, 1,3 éq.) sont mis en suspension dans le dichlorométhane (212 g). Le milieu réactionnel est porté à reflux puis une solution de triphosgène (9,55g, 0,35 éq.) dans le dichlorométhane (64 g) est coulée. Après des lavages liquide/liquide de la phase organique à l'eau, le dichlorométhane est évaporé pour conduire à la N-[1-(S)-éthoxycarbonyl-butyl]-(S)-alanine activée de formule (III) (22 g). Celle-ci est ensuite mise en solution dans l'acétonitrile (180 g). La solution est coulée sur le (2S, 3aS, 7aS)-2-carboxyperhydroindole (15 g, 1 éq.) et le milieu réactionnel est agité pendant environ 5 h en présence de triéthylamine (9,15 g, 1 éq.) à une température inférieure à 10°C puis clarifié sur un filtre pour obtenir une solution limpide. Le sel de L-arginine du périndopril est obtenu par ajout de L-arginine (14,5 g, 0,90 éq.) en suspension dans le DMSO (180 g) à la température de 50°C. Après un temps de contact d'environ 5h, le milieu est amorcé avec 2% de forme delta puis agité une nuit à 50°C et filtré en cellule de filtration.
Le perindopril L-arginine (43 g) est obtenu avec un rendement de 89 % par rapport au (2S, 3aS, 7aS)-2-carboxyperhydroindole (amorce déduite). La qualité HPLC du produit isolé est supérieure à 99 %.
Le débit de filtration des jus mères est d'environ 2000 kg/h/m².

### EXEMPLE 7 : Sel de L-arginine du périndopril - à partir du périndopril tert-butylamine; dans un mélange ternaire acétonitrile/ diméthylsulfoxyde / toluène 30/40/30, avec amorce

Mettre le périndopril tert-butylamine (103 g, 1 éq.) et du chlorure de sodium (5,84 g) en suspension dans le toluène (268 g, d = 0,867). Agiter à température ambiante.
Ajouter une solution d'acide chlorhydrique (57,8 mL, # 4 N, 1 éq.). Agiter 40 minutes à température ambiante. Séparer la phase toluénique de la phase aqueuse.
Laver la phase aqueuse par du toluène (2 x 90 g, d = 0,867).
A ce stade, le périndopril se trouve en solution dans le toluène à la concentration de 16% m/m. La L-arginine (36,6 g, 0,90 éq.) et le diméthylsulfoxyde (566 g, d = 1,100) sont ajoutés et le milieu réactionnel est chauffé à 50°C pendant 5h. De l'acétonitrile (405 g, d = 0,787) est ajouté et le milieu réactionnel est amorcé avec 2 % massique de périndopril L-arginine sous forme delta (composé de l'Exemple 1).
La suspension est agitée pendant 17h puis la température est amenée à 30°C en 30 minutes. Après 2h d'agitation, le produit est isolé par filtration. Le gâteau est lavé et séché.
Le périndopril L-arginine (95 g) est obtenu avec un rendement de 75 % (amorce déduite) par rapport au périndopril tert-butylamine. La qualité HPLC du produit isolé est supérieure à 99,8 %.
Le débit de filtration des jus mères est d'environ 4000 kg/h/m².

### EXEMPLE 8 : Sel de L-arginine du périndopril - à partir du périndopril (acide libre), dans un mélange ternaire acétonitrile/DMSO/toluène 30/40/30, avec amorce

Le périndopril lyophilisé (8,3 g, 1 éq.) est mis en solution dans un mélange de toluène (43 g) et de DMSO (55 g). La L-arginine (3,9 g, 1 éq.) est introduite en suspension dans l'acétonitrile (40 g) et l'ensemble est chauffé à 50°C.
Le milieu réactionnel est amorcé avec 3 % de périndopril arginine sous la forme delta et la suspension est agitée à 50°C pendant 22h.
Le produit est isolé par filtration. Le périndopril L-arginine (9 g) est obtenu avec un rendement de 73 % (amorce déduite) par rapport au périndopril. La qualité HPLC du produit isolé est supérieure à 99 %.

### EXEMPLE 9 : Sel de L-arginine du périndopril à partir du périndopril tert-butylamine, dans un mélange binaire acétate d'éthyle/DMSO 55/45, avec amorce

Dans un réacteur charger le périndopril erbumine (200g, 1 éq.), du méthyl tétrahydrofuranne (700g) et de l'acide méthanesulfonique (43,5g, 1 éq.).
Filtrer l'insoluble et ajouter sur la solution la L-arginine (78,8g, 1 éq) et du DMSO (500 g).
Distiller le méthyl tétrahydrofuranne et chauffer à 70°C pendant 1h.
Ajouter de l'acétate d'éthyle (600 g) et amorcer avec 2 % de périndopril arginine de forme cristalline delta.
Refroidir à 25°C en 4 heures, filtrer et laver le produit avec un mélange acétate d'éthyle/DMSO.
Le périndopril L-arginine (217 g amorce déduite) est obtenu avec un rendement de 88 % par rapport au périndopril. La qualité HPLC du produit isolé est supérieure à 99 %.
Le débit de filtration des jus mères est supérieur à 1500 kg/h/m².

### EXEMPLE 10 : Sel de L-arginine du périndopril - à partir du périndopril (acide libre), dans un mélange ternaire acétonitrile/DMSO/toluène 45/50/5, avec amorce

Dans un réacteur, charger le périndopril lyophilisé (30 g, 1 éq.), le DMSO (100 g) et la L-arginine (13,8 g, 1 éq.). Le milieu est chauffé à 70°C pendant 2h puis un mélange d'acétonitrile (90 g) et de toluène (10 g) est ajouté. Le milieu réactionnel est amorcé avec 2 % de périndopril arginine de forme cristalline delta et la suspension est agitée pendant 2h à 70°C.

Refroidir à 25°C en environ 4 heures, filtrer et laver le produit avec de l'acétonitrile et du DMSO.
Le périndopril L-arginine (41 g amorce déduite) est obtenu avec un rendement de 92 % par rapport au périndopril.
La qualité HPLC du produit isolé est supérieure à 99 %. Le débit de filtration des jus mères est supérieur à 1500 kg/h/m².

### EXEMPLE comparatif A: Adaptation du mode de l'Exemple 3 de EP 1 279 665 à l'obtention du sel de L-arginine du périndopril

Le réacteur est préalablement refroidi à 0°C. La N-[1-(S)-éthoxycarbonyl-butyl]-(S)-alanine (80 g - 1 éq.) et le dichlorométhane (1325 g, d = 1,325) sont introduits. Le N, N'-carbonyldiimidazole (71,5 g - 1,2 éq.) et 0,336 L de dichlorométhane sont additionnés sur le milieu. La température du milieu est préalablement amenée à -5°C avant ajout du (2S, 3aS, 7aS)-2-carboxyperhydroindole (81 g - 1,3 éq.). Après 2h30 de temps de contact, le milieu est mis à sec puis repris avec de l'eau (1200 g, d = 1,00). Après acidification de la solution aqueuse (avec 185 ml d'une solution d'HCl 4N), la solution est extraite avec du dichlorométhane (2517,5 g, d = 1,325) et la phase aqueuse est saturée en NaCl. La phase organique est mise à sec et le résidu de mise à sec repris avec de l'acétate d'éthyle (1176,6 g, d = 0,902). La L-arginine est alors ajoutée (68 g- 1,06 éq.) et le milieu est maintenu sous agitation la nuit à 50°C.
La filtration du milieu se révèle impossible car le solide est d'aspect collant. Le solide est extrait du réacteur par démontage de la cuve. Rendement (sur titre)= 1,6%.

### EXEMPLE comparatif B : selon l'Exemple 6 de la demande de brevet WO 2009/157018

Le périndopril (30g) et la L-arginine (13,8g) sont mis en suspension dans le toluène (130 g, d = 0,867) à température ambiante. Le milieu est placé au reflux pendant une heure. Puis de l'acétonitrile (1180,5 g, d= 0,787) est additionné à 80°C. Après maintien d'une heure sous agitation à cette température, la suspension est filtrée en cellule sous 0,3 bar d'azote.

Le débit moyen de filtration des jus mères a été mesuré à 100 kg/h/m². Le produit isolé est d'aspect collant et de couleur rose pâle. Le rendement pondéral est de 46,5%. La qualité HPLC du produit isolé est de l'ordre de 83 %.

## Revendications

1. Procédé de préparation du sel de L-arginine du périndopril, de formule (I) : 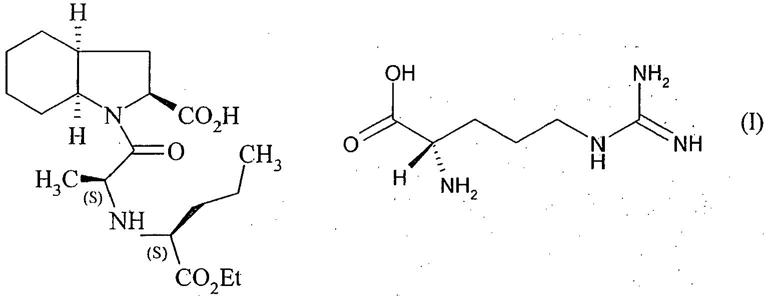 par réaction entre le périndopril et la L-arginine dans un système de solvants choisi parmi :
un mélange binaire d'acétonitrile et de diméthylsulfoxyde,
un mélange binaire d'acétate d'éthyle et de diméthylsulfoxyde,
un mélange ternaire d'acétonitrile, de diméthylsulfoxyde et de toluène,
à une température de 10 à 100°C,
suivie de l'isolement par filtration du sel de L-arginine ainsi obtenu.

2. Procédé de préparation du sel de L-arginine du périndopril selon la revendication 1, dans lequel le périndopril est obtenu par réaction de la N-1-(S)-éthoxycarbonyl-butyl]-(S)-alanine de formule (II) : 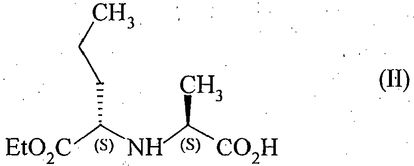 avec un agent d'activation de formule X₂C=O dans laquelle X représente un groupe partant,
dans un solvant organique ou système de solvants organiques,
à une température de -20 à 80°C,
suivie de la réaction du composé intermédiaire de formule (III) ainsi obtenu : 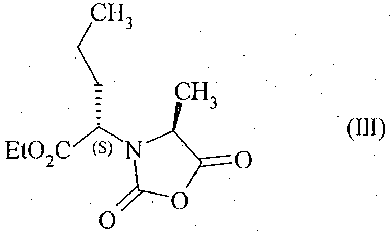 avec le (2S, 3aS, 7aS)-2-carboxyperhydroindole,
à une température de 0 à 80°C,
puis le périndopril ainsi obtenu est mis en réaction avec la L-arginine selon le procédé de la revendication 1.

3. Procédé de préparation du sel de L-arginine du périndopril selon la revendication 2, où l'agent d'activation est le N, N'-carbonyldiimidazole, le phosgène, le triphosgène, le (1,1'-carbonyldi(1,2,4-triazole) ou le di(N-succinimidyl)carbonate.

4. Procédé de préparation du sel de L-arginine du périndopril selon la revendication 3, où l'agent d'activation est le N, N'-carbonyldiimidazole et la quantité de N, N'-carbonyldiimidazole est comprise entre 0,8 et 1,2 moles par mole de N-[1-(S)-éthoxycarbonyl-butyl]-(S)-alanine.

5. Procédé de préparation du sel de L-arginine du périndopril selon l'une quelconque des revendications 2 à 4, où la quantité de (2S, 3aS, 7aS)-2-carboxyperhydroindole est comprise entre 0,8 et 1,2 moles par mole de N-[1-(S)-éthoxycarbonyl-butyl]-(S)-alanine.

6. Procédé de préparation du sel de L-arginine du périndopril selon la revendication 1, dans lequel le périndopril est obtenu par désalification du périndopril tert-butylamine par action d'un acide, puis le périndopril ainsi obtenu est mis en réaction avec la L-arginine selon le procédé de la revendication 1.

## Patentansprüche

1. Verfahren zur Herstellung des L-Argininsalzes von Perindopril der Formel (I): 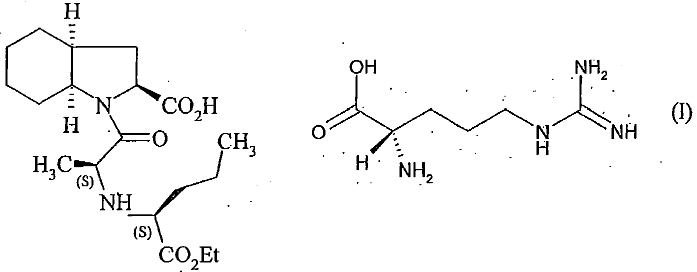 durch Umsetzung von Perindopril mit L-Arginin in einem Lösungsmittelsystem ausgewählt aus:
einer binären Mischung aus Acetonitril und Dimethylsulfoxid,
einer binären Mischung aus Ethylacetat und Dimethylsulfoxid,
einer ternären Mischung aus Acetonitril, Dimethylsulfoxid und Toluol,
bei einer Temperatur von 10 bis 100 °C,
gefolgt von der Isolierung des in dieser Weise erhaltenen L-Argininsalzes durch Filtration.

2. Verfahren zur Herstellung des L-Argininsalzes von Perindopril nach Anspruch 1, gemäß dem Perindopril erhalten wird durch Umsetzung von N-[1-(S)-Ethoxycarbonyl-butyl]-(S)-alanin der Formel (II): 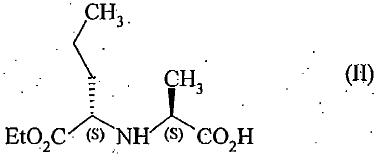 mit einem Aktivierungsmittel der Formel X₂C=O, worin X eine austretende Gruppe bedeutet,
in einem organischen Lösungsmittel oder einem System aus organischen Lösungsmitteln,
bei einer Temperatur von -20 bis 80 °C,
gefolgt von der Umsetzung der in dieser Weise erhaltenen Zwischenverbindung der Formel (III): 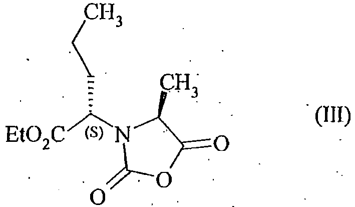 mit (2S, 3aS, 7aS)-2-Carboxyperhydroindol,
bei einer Temperatur von 0 bis 80 °C,
wonach das in dieser Weise erhaltene Perindopril nach dem Verfahren von Anspruch 1 mit L-Arginin umgesetzt wird.

3. Verfahren zur Herstellung des L-Argininsalzes von Perindopril nach Anspruch 2, worin das Aktivierungsmittel N,N'-Carbonyldiimidazol, Phosgen, Triphosgen, (1,1'-Carbonyldi(1,2,4-triazol)) oder Di(N-succinimidyl)-carbonat ist.

4. Verfahren zur Herstellung des L-Argininsalzes von Perindopril nach Anspruch 3, worin das Aktivierungsmittel N,N'-Carbonyldiimidazol ist und die Menge von N,N'-Carbonyldiimidazol zwischen 0,8 und 1,2 Mol pro Mol N-[1-(S)-Ethoxycarbonyl-butyl]-(S)-alanin beträgt.

5. Verfahren zur Herstellung des L-Argininsalzes von Perindopril nach einem der Ansprüche 2 bis 4, worin die Menge von (2S, 3aS, 7aS)-2-Carboxyperhydroindol zwischen 0,8 und 1,2 Mol pro Mol N-[1-(S)-Ethoxycarbonyl-butyl]-(S)-alanin beträgt.

6. Verfahren zur Herstellung des L-Argininsalzes von Perindopril nach Anspruch 1, worin Perindopril erhalten wird durch Entsalzung von Perindopril-tert.-butylamin durch Einwirkung einer Säure, wonach das in dieser Weise erhaltene Perindopril nach dem Verfahren von Anspruch 1 mit L-Arginin umgesetzt wird.

## Claims

1. Process for the preparation of perindopril L-arginine salt of formula (I): 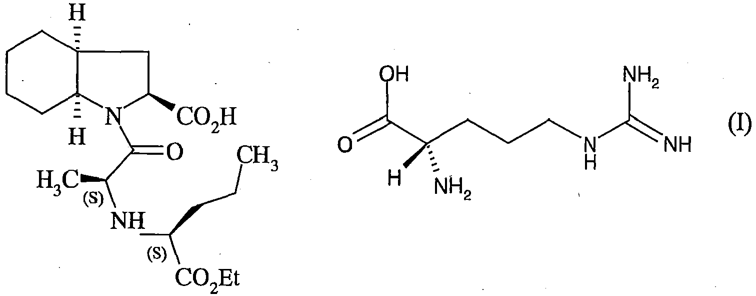 by means of reaction between perindopril and L-arginine in a solvent system selected from:
a binary mixture of acetonitrile and dimethyl sulphoxide,
a binary mixture of ethyl acetate and dimethyl sulphoxide,
a ternary mixture of acetonitrile, dimethyl sulphoxide and toluene,
at a temperature from 10 to 100°C,
followed by isolation by means of filtration of the L-arginine salt thereby obtained.

2. Process for the preparation of perindopril L-arginine salt according to claim 1, wherein the perindopril is obtained by reaction of N-[1-(S)-ethoxycarbonyl-butyl]-(S)-alanine of formula (II): 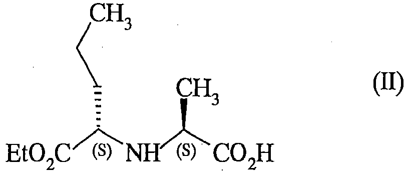 with an activation agent of formula X₂C=O wherein X represents a leaving group,
in an organic solvent or a system of organic solvents,
at a temperature from -20 to 80°C,
followed by reaction of the intermediate compound of formula (III) thereby obtained: 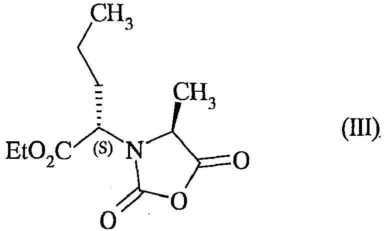 with (2S, 3aS, 7aS)-2-carboxyperhydroindole,
at a temperature from 0 to 80°C,
and then the perindopril thereby obtained is reacted with L-arginine in accordance with the process of claim 1.

3. Process for the preparation of perindopril L-arginine salt according to claim 2, wherein the activation agent is N,N'-carbonyldiimidazole, phosgene, triphosgene, (1,1'-carbonyldi(1,2,4-triazole) or di(N-succinimidyl) carbonate.

4. Process for the preparation of perindopril L-arginine salt according to claim 3, wherein the activation agent is N,N'-carbonyldiimidazole and the amount of N,N'-carbonyldiimidazole is between 0.8 and 1.2 moles, inclusive, per mole of N-[1-(S)-ethoxycarbonyl-butyl]-(S)-alanine.

5. Process for the preparation of perindopril L-arginine salt according to any one of claims 2 to 4, wherein the amount of (2S, 3aS, 7aS)-2-carboxyperhydroindole is between 0.8 and 1.2 moles, inclusive, per mole of N-[1-(S)-ethoxycarbonyl-butyl]-(S)-alanine.

6. Process for. the preparation of perindopril L-arginine salt according to claim 1, wherein the perindopril is obtained by desaltification of perindopril tert-butylamine by the action of an acid, and then the perindopril thereby obtained is reacted with L-arginine in accordance with the process of claim 1.
